# EUROPEAN PATENT APPLICATION

(11) **EP 3 928 708 A1**
(43) Date of publication of application: **29.12.2021**
(21) Application number: 20182459.6
(22) Date of filing: 26.06.2020
(51) Int. Cl.: A61B 6/10, A61B 6/00

(54) **MOBILE IMAGING SYSTEM CAPABLE OF DETECTING OBSTACLES**

(71) Applicant: Koninklijke Philips N.V., 5656 AG Eindhoven (NL)
(72) Inventor: DONGARE, Mukund Dhondiram, 5656 AE Eindhoven (NL)
(74) Representative: Philips Intellectual Property & Standards

(57) **Abstract**

The present invention relates to a mobile imaging system, such as mobile C-arm X-ray system, mobile ultrasound system, mobile patient monitoring system. The mobile imaging system comprises one or more cameras arranged on the front end of the mobile stand of the mobile imaging system. The one or more cameras have a field of view that covers one or more regions in front of the mobile stand. Indicators (e.g. color codes) may be generated to overlay the camera image in such a way that they can assist the user in better appreciating the distances on the floor from the camera and so the risk of an obstacle.

## Description

### FIELD OF THE INVENTION

The present invention relates in general to a mobile imaging system, and in particular to a mobile imaging system, a computer-implemented method for controlling mobile imaging system, a computer program element, and a computer readable medium.

### BACKGROUND OF THE INVENTION

Medical imaging apparatuses are often used in hospitals as an aid for obtaining information for making a diagnosis. For a flexible use of the medical imaging apparatuses and increased economic efficiency, some medical imaging apparatus, such as X-ray apparatus, ultrasound apparatus, or patient monitoring apparatus, are constructed as mobile apparatuses. Such mobile imaging apparatuses may thus be moved between individual facilities to locations where an examination, e.g. radiological examination, is about to be carried out. When moving a mobile imaging apparatus, a field of view of the operator may be blocked by a mobile stand and a display device of the mobile imaging apparatus. Accordingly, there is a risk that the moving mobile imaging apparatus may collide against an obstacle located within a blind spot of the operator. For a mobile C-arm X-ray system, there is also a risk that the moving C-arm may collide against Operation Theater (OT) equipment.

### SUMMARY OF THE INVENTION

There may be a need to provide an improved mobile imaging apparatus.

The object of the present invention is solved by the subject-matter of the independent claims, wherein further embodiments are incorporated in the dependent claims. It should be noted that the following described aspects of the invention apply also for the mobile imaging system, the computer-implemented method, the computer program element, and the computer readable medium.

According to a first aspect of the present invention, there is provided a mobile imaging system. The mobile imaging system comprises:
- an imaging arrangement for acquiring image data;
- an internal power source for supplying power to the mobile imaging system;
- a display arrangement with at least one display device;
- a mobile stand bearing the imaging arrangement, the internal power source, and the display arrangement;
- a camera arrangement with at least one camera arranged on a frond end of the mobile stand to have a field of view that covers a region in front of the mobile stand; and
- a controller configured to receive a control signal indicative of a mode change and to control a user interface displayed on the at least one display device to switch between a moving assistance mode and a control mode according to the control signal.

In the moving assistance mode, the user interface is configured to receive a signal from the camera arrangement and to display a camera image showing the region in front of the mobile stand for assisting in moving the mobile stand; and

In the control mode, the user interface is configured to enable a control of parameters and settings of the imaging arrangement for acquiring image data and/or a display of an image acquired by the imaging arrangement.

In other words, a mobile imaging system, such as mobile C-arm X-ray system, mobile ultrasound system, mobile patient monitoring system, is proposed that comprises one or more cameras arranged on the front end of the mobile stand of the mobile imaging system. The one or more cameras have a field of view that covers one or more regions in front of the mobile stand. Indicators (e.g. color codes) may be generated to overlay the camera image in such a way that they can assist the user in better appreciating the distances on the floor from the camera and so the risk of an obstacle. For example, indicators may take into account the design of the C-arm and in particular its height for user to travel and park the mobile C-arm X-ray system. The one or more cameras may be powered by an internal power source of the mobile imaging system. For example, most mobile C-arm X-ray systems have battery packs, which are used for a system with a high-voltage and/or high-current capability. The battery packs are of 300 volts, which can be used to enable the camera viewing system, especially when the C-arm is in off mode. Another example of the internal power source may be a fuel cell. Unlike a battery, it does not store chemical or electrical energy; electrical energy is provided by a chemical reaction.

Accordingly, the proposed mobile imaging system may improve the system maneuverability and may have at least one of the following advantages:
overcoming the safety issue;
preventing OT equipment damage;
preventing the damage of the mobile imaging system;
having the ability to manure easily and to detect the direction of a potential collision;
predicting the exact distance and/or height which can be used for repositioning of C-arm, e.g. with the help of distance markings and/or height markings (e.g. color codes);
no extra cost of battery maintenance with the same battery pack for powering up the imaging arrangement (e.g. X-ray generation system), the camera arrangement, and the display arrangement; and
maximum use of existing hardware and minimum change in the mobile imaging system.

According to an embodiment of the present invention, wherein the control signal is obtainable from the user interface.

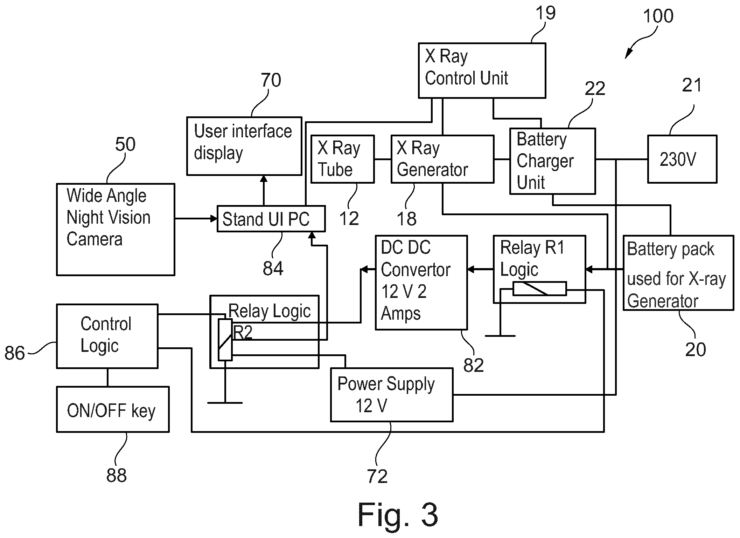

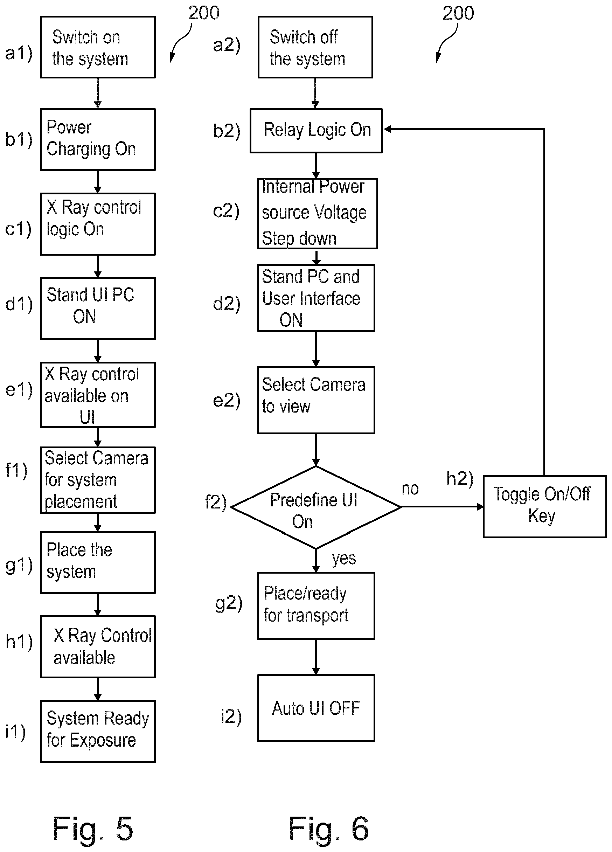

For example, an operator can select the moving assistance mode on the user interface (UI) for system placement to avoid collision with an operating table and OT equipment. Once the system is placed, the operator can select the control mode, which will give access to all the controls and system will be ready for exposures. This will be explained hereafter and in particular with respect to the embodiments illustrated in Figs. 3 and 5.

According to an embodiment of the present invention, the control signal is configured to be generated in response to a change of power state of the imaging arrangement.

In an example, if the imaging arrangement, such as X-ray generation system, is switched off, the control logic for the imaging arrangement is off. There is no communication between the user interface and the control logic for the imaging arrangement. Accordingly, a control of parameters and settings of the imaging arrangement is disabled. Therefore, the user interface then displays the camera image for the user to travel and park the mobile imaging system.

In an example, if the imaging arrangement is switched on, the control logic for the imaging arrangement is also on. There is a communication between the user interface and the control logical for the imaging arrangement. Accordingly, a control of parameters and settings of the imaging arrangement is enabled, which will be displayed on the screen to allow the user to control the parameters and settings of the imaging arrangement or to view an acquired image. This will be explained hereafter and in particular with respect to the embodiments illustrated in Figs. 3 and 6.

According to an embodiment of the present invention, the control signal is configured to be generated to switch the user interface to the moving assistance mode, when a power state of the imaging arrangement is changed from an on-state to an off-state.

Using the mobile C-arm X-ray system as an example, the operator turns the X-ray machine off at the end of the imaging session, which may trigger the user interface to switch from the control mode to the moving assistance mode. In the moving assistance mode, the camera image is displayed showing e.g. an area below the operating table for facilitating moving the imaging arrangement.

According to an embodiment of the present invention, in the moving assistance mode, the controller is configured to receive a signal indicative of distance information in front of the mobile stand and/or height information in front of the mobile stand. The user interface is configured to display the camera image together with a distance marking projected onto a ground indicative of the distance information and/or a height marking indicative of the height information.

In an example, the signal may be received from a distance sensor, such as ultrasound sensor, or radar sensor.

In an example, the signal may be received from the camera arrangement. For example, the camera arrangement may be a stereo vision system, i.e. a computer vision system that is based on stereoscopic ranging techniques to calculate the distance.

In an example, a distance marking, e.g. indicators like color codes, may be generated to overlay the camera image in such a way that they can assist the user in better appreciating the distances on the floor from the camera and so the risk of an obstacle. For example, indicators may take into account the design of the C-arm and in particular its height for user to travel and park the mobile C-arm X-ray system.

In an example, a height marking, e.g. indicators like color codes, may be generated to overlay the camera image in such a way that they can assist the user in better appreciating the height of e.g. C-arm with respect to the obstacle, e.g. operating table, OT equipment, for precisely placing the C-arm. With the distance marking and/or the height marking, the exact distance and/or height which can be used for repositioning of C-arm may be predicted.

According to an embodiment of the present invention, the display arrangement is configured to be powered by the internal power source for a predefined time, when a power state of the imaging arrangement is an off-state.

When a power state of the imaging arrangement is an off-state, the display arrangement and the camera arrangement are powered by the internal power source. As the user interface screen will be live for pre-defined time, e.g. 5 minutes, 10 minutes, etc., the rapid battery drain, for example, may be avoided.

The user interface may be switched on with the help of e.g. an ON/OFF key toggle for better control in power off mode, as there is no X-ray control screen available.

According to an embodiment of the present invention, the display arrangement comprises:
(i) a single display device with an image screen for displaying the camera image, and the parameters and settings of the imaging arrangement; or
(ii) at least two display devices with at least two image screens for displaying the camera image, and the parameters and settings of the imaging arrangement.

According to an embodiment of the present invention, the display arrangement is arranged on at a back end of the mobile stand.

According to an embodiment of the present invention, the camera arrangement is configured to be supplied with power from the internal power source.

For example, in a mobile C-arm X-ray system, the same battery pack may be used for powering up the imaging arrangement and the camera arrangement. For example, as most mobile C-arm X-ray systems have a battery pack, no additional battery is required for powering the camera arrangement. Accordingly, no extra cost of battery maintenance is required.

According to an embodiment of the present invention, the camera arrangement comprises a wide-angle night version camera.

A night version camera may be beneficial for addressing different light conditions inside and outside the OT.

Instead of or in additional to a wide-angle night version camera, the camera arrangement may comprise a plurality of night version cameras having fields of view that cover different regions in front of the mobile stand.

According to an embodiment of the present invention, the mobile imaging system further comprises an electromagnetic brake configured to be released, when at least one camera in the camera arrangement is on and/or the user interface is in the moving assistance mode, as long as any alert signal representative of a presence of an obstacle within a predefined distance range is not received.

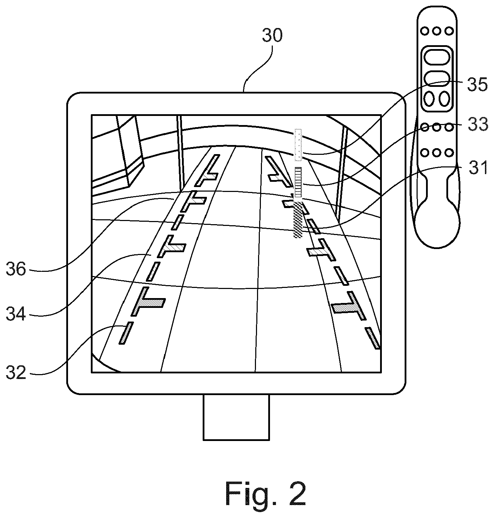

In other words, the electromagnetic brake may be designed in such a manner that the brake is released, when at least one camera in the camera arrangement is on and/or the user interface is in the moving assistance mode. In other words, the mobile stand may be allowed to travel, if the camera image is displayed showing the region in front of the mobile stand for assisting in moving the mobile stand. However, if an obstacle is detected within a predefined distance range, e.g. a high-risk space indicated with a red color shown in Fig. 2 that is very close to the mobile stand, the electromagnetic brake may be activated to automatically bring the mobile imaging system to a halt for avoiding colliding against the obstacle.

Due to manual movement required in a power fail condition, the minimum force requirement as per IEC medical standards, which makes break mechanism more complex for all the axis. This can be avoided by using the power from the internal power source.

According to an embodiment of the present invention, the imaging arrangement comprises at least one of X-ray, ultrasound, or patient monitoring device.

According to a second aspect of the present invention, there is provided a method for controlling mobile imaging system that comprises an imaging arrangement for acquiring image data, an internal power source for supplying power to the mobile imaging system, a display arrangement with at least one display device; a mobile stand bearing the imaging arrangement, the internal power source, and the display arrangement, and a camera arrangement with at least one camera arranged on a frond end of the mobile stand to have a field of view that covers a region in front of the mobile stand, the method comprising:
- receiving, by a controller, a control signal indicative of a mode change; and
- controlling, by the controller, a user interface to switch between a moving assistance mode and a control mode according to the control signal;
wherein, in the moving assistance mode, the user interface receives a signal from the camera arrangement and displays a camera image showing the region in front of the mobile stand; and
wherein, in the control mode, the user interface enables a control of parameters and settings of the imaging arrangement for acquiring image data.

According to a third aspect of the present invention, there is provided a computer program element for controlling a mobile imaging system according to the first aspect and any associated example, which when being executed by a processor is configured to carry out the method according to the second aspect and any associated example.

According to another aspect of the present invention, there is provided a computer readable medium having stored thereon the program element according to the third aspect.

As used herein, the term "controller" is used generally to describe various apparatus relating to the operation of an apparatus, system, or method. A controller can be implemented in numerous ways (e.g., such as with dedicated hardware) to perform various functions discussed herein. A "processor" is one example of a controller which employs one or more microprocessors that may be programmed using software (e.g., microcode) to perform various functions discussed herein. A controller may be implemented with or without employing a processor, and also may be implemented as a combination of dedicated hardware to perform some functions and a processor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs).These and other aspects of the present invention will become apparent from and be elucidated with reference to the embodiments described hereinafter.

In various implementations, a processor or controller may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects of the present disclosure discussed herein. The terms "program" or "computer program" are used herein in a generic sense to refer to any type of computer code (e.g., software or microcode) that can be employed to program one or more processors or controllers.

The term "internal power source" is used generally to describe various power sources that are located in the same housing as the mobile imaging system that they power. Examples of the internal power source may include, but are not limited to, batteries and fuel cells. In an example, the internal power source may be a built-in power source - that is, the internal power supply may be an integral and permanent part of the mobile imaging system. In another example, the internal power source may be a replaceable power source. The term "external power source" is used generally to describe various power sources that are contained in a housing separate from the mobile imaging system they power.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 schematically illustrates an exemplary mobile imaging system.
Fig. 2 illustrates an exemplary camera image with distance markings and height markings.
Fig. 3 schematically illustrates a block diagram of a further exemplary mobile imaging system.
Fig. 4 illustrates a flowchart of an example of the computer-implemented method.
Fig. 5 illustrates a flowchart of another example of the computer-implemented method.
Fig. 6 illustrates a flowchart of a further example of the computer-implemented method.

### DETAILED DESCRIPTION OF EMBODIMENTS

Although the discussion below is focused on a mobile C-arm X-ray system as an example of the mobile imaging system, one skilled in the art would understand that the approach of the present disclosure is also useful for other kinds of mobile imaging systems, such as mobile ultrasound system, mobile patient monitoring system, etc.

Using a mobile C-arm X-ray system as an example, the mobile C-arm X-ray system is a piece of medical equipment used in an OT for various surgical procedures. It comprises a robotic/manual C-arm, whose motion is controlled by a human operator. During surgery, the C-arm moves around a patient lying on the operation table. There is a risk that the moving C-arm may collide against OT equipment. Additionally, the mobile C-arm X-ray system may be moved from OT to OT or parking. As the field of view may be blocked by C-Arm and the display devices, the user may be unable see through C-arm stand while moving the mobile system. This may cause damage to FD/II, monoblock, and/or other equipment around.

Towards this end, according to a first aspect of the present disclosure, there is provided a mobile imaging system 100. The mobile imaging system 100 comprises:
- an imaging arrangement 10 for acquiring image data;
- an internal power source 20 for supplying power to the mobile imaging system;
- a display arrangement 30 with at least one display device;
- a mobile stand 40 bearing the imaging arrangement, the internal power source, and the display arrangement;
- a camera arrangement 50 with at least one camera arranged on a frond end of the mobile stand to have a field of view that covers a region in front of the mobile stand; and
- a controller 60 configured to receive a control signal indicative of a mode change and to control a user interface 70 displayed on the at least one display device to switch between a moving assistance mode and a control mode according to the control signal;
wherein, in the moving assistance mode, the user interface is configured to receive a signal from the camera arrangement and to display a camera image showing the region in front of the mobile stand for assisting in moving the mobile stand; and
wherein, in the control mode, the user interface is configured to enable a control of parameters and settings of the imaging arrangement for acquiring image data.

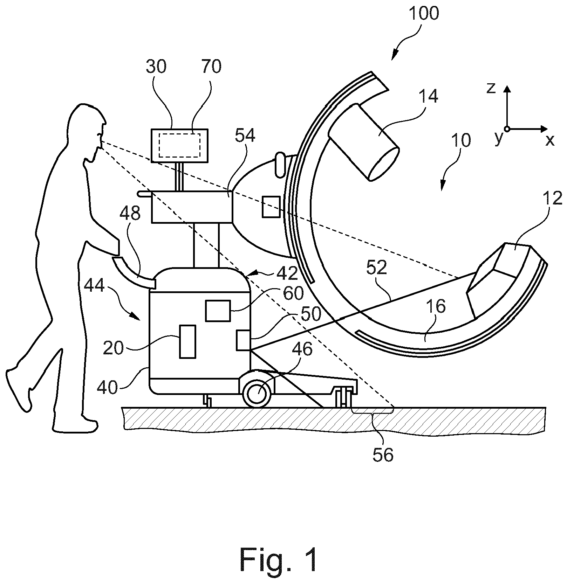

Fig. 1 schematically illustrates an exemplary mobile imaging system 100 according to the first aspect of the present disclosure. In this example, the exemplary mobile imaging system 100 is a mobile C-arm X-ray system. One skilled in the art would understand that the method of the present disclosure is also useful for other mobile imaging systems, such as ultrasound, or patient monitoring system.

The mobile C-arm X-ray system 100 comprises an imaging arrangement 10. The imaging arrangement 10 comprises an X-ray source 12 and an X-ray detector 14 arranged on a C-arm 16. The C-arm 16 may be moveable in a horizontal direction and/or in a vertical direction. The C-arm 16 may be rotatable around a horizontal axis and/or around a vertical axis.

The mobile C-arm X-ray system 100 further comprises an internal power source 20 for supplying power to the mobile imaging system 100. The internal power source 20 may be e.g. a fuel cell, a battery, etc. To facilitate the understanding of the system, the battery will be described henceforth as an example of the internal power source. The battery 20 may also be referred to as energy storage unit. The battery 20 may include e.g. a lithium-ion battery pack. The battery 20 may be used for the power supply for all the device functions. For example, the device functions may include the operation of the X-ray source 12. For supplying energy to the X-ray source 12, a high electric voltage of e.g. 300 volts may be achieved by series connection of a plurality of individual cells. For example, the battery may have a high-current capability. A plurality of storage cells may be connected in parallel in order to generate a strong enough current to operate the X-ray source 12. For example, the device functions may include the operation of the C-arm 14. For example, the C-arm 16 may be powered by the battery 20 to perform a translational and/or a rotational movement.

The mobile C-arm X-ray system 100 further comprises a display arrangement 30. The display arrangement 30 may comprise one or more display devices.

In an example, the display arrangement 30 may comprise a single display device. In another example, the display arrangement 30 may comprise two or more display devices. Each display device has an image screen, where a user interface is displayed.

In an example, the display arrangement 30 may be configured to be powered by the battery 20 for a predefined time, e.g. for 5 minutes, 10 minutes, etc., when a power state of the imaging arrangement 10 is an off-state. The display arrangement 30 will be on for a pre-defined time to avoid battery drain out. To turn the display arrangement 30 on, an ON/OFF key toggle may be used.

The mobile C-arm X-ray system 100 further comprises a mobile stand 40 that bears the imaging arrangement 10, the battery 20, and the display arrangement 30. The mobile stand 40 has a front end 42 and a back end 44 opposite to the front end 42. The front end 42 may give storage to a storage space, such as drawers, etc. The mobile stand 40 typically has wheels 46. In an example, at a position close to the back end 44, one or more display devices of the display arrangement 30 may be mounted thereon. The back end 44 may have a handle 48 that allows an operator to move the mobile stand 40, e.g. to move the mobile C-arm X-ray system 100 out of an OT or parking.

Due to the position and height of the C-arm 16, the operator may be unable to see through the C-arm 16. Therefore, it may become difficult for maneuverability, which requires constant attention, failure of which may cause collision and damage of the mobile C-arm X-ray system. For example, Fig. 1 illustrates a line of sight 54 and a blind spot 56 of the operator, due to which there is possibility of collision, which may cause damage to the mobile C-arm X-ray system 100 or other OT equipment. Similarly, while placing the mobile C-arm X-ray system 100 on an operating table, the user view may be blocked due to drapes. There is thus possibility of colliding the mobile C-arm X-ray system to the operating table.

To solve the above-mentioned problems, a camera arrangement 50 is provided and arranged on a front end 42 of the mobile stand 40. The camera arrangement 50 comprises at least one camera to have a field view 52 that covers a region in front of the mobile stand 40. Preferably, the field of view may cover the blind spot 56 and an area below the operating table for easing the placement of the imaging arrangement 10. In an example, the camera arrangement 50 may have a plurality of cameras having fields of view that cover different regions in front of the mobile stand 40. The multiple camera combination may be placed for accurate placement, such as placement of C-arm below an operating table in the OT. In an example, the camera arrangement 50 may comprise a wide-angle night version camera for better adapting to different light conditions inside and outside the OT.

The mobile C-arm X-ray system 100 further comprises a controller 60. The controller 60 is configured to receive a control signal indicative of a mode change and to control a user interface 70 displayed on the at least one display device to switch between a moving assistance mode and a control mode according to the control signal.

In the moving assistance mode, the user interface 70 is configured to receive a signal from the camera arrangement and to display a camera image showing the region in front of the mobile stand for assisting in moving the mobile stand.

An exemplary camera image is illustrated in Fig. 2. In this example, the controller 60 is configured to obtain a signal indicative of distance information in front of the mobile stand 40.

In an example, the signal may be received from one or more distance sensors. For example, the one or more distance sensors may be designed as ultrasound sensors, which are sent out the ultrasound signal, receive the reflected ultrasound signal and determine a distance from the mobile stand to obstacles next to the mobile stand from the running time of the ultrasound signal. Instead of ultrasonic sensors, sensors can also be used which emit other wave signals, such as electromagnetic waves, for example radar waves.

In another example, the signal may be obtained from the camera arrangement 50 that comprises at least two cameras forming a stereo vision system. The stereo vision system is a computer vision system that is based on stereoscopic ranging techniques to calculate the distance.

The user interface 70 may be configured to display the camera image together with a distance marking projected onto a ground to show the distance information.

In an example, as illustrated in Fig. 2, the distance information is indicated to a user via a color code. For example, a red color 32 may indicate a high-risk space that is very close to the mobile stand and there is a high risk of collision between the mobile C-arm X-ray system and an object located within the high-risk space. For example, a yellow color 34 may indicate a low-risk space that is relatively far away from the mobile stand and there is less risk of collision between the mobile C-arm X-ray system 100 and an object located within the low-risk space. For example, a green color 36 may indicate a safe space that is far away from the mobile stand and there is no risk of collision between the mobile C-arm X-ray system and an object located within the safe space.

Alternatively or additionally, the user interface 70 may be configured to display the camera image together with a height marking with respect to an obstacle.

In an example, as illustrated in Fig. 2, the height markings 31, 33, 35 may be projected onto the ground indicative of the height information with respect to the ground. In an example, each height marking may represent a height of e.g. 10 cm. In another example, each height marking may represent a height of e.g. 15 cm. In a further example, each height marking may represent a height of e.g. 20 cm. If there is an obstacle in the camera image, the height information may shows the height in relation to the obstacle. For example, if the camera image shown in Fig. 2 shows an area below an operating table, the indicator 35 may indicate a high-risk height that is very close to the operating table and there is a high risk of collision. The indicator 33 may indicate a low-risk height that is relatively far away from the operating table and there is low risk of collision. The indicator 3 1 may indicate a safe height that is far away from the operating table.

In the control mode, the user interface 70 is configured to enable a control of parameters and settings of the imaging arrangement for acquiring image data or enable a display of an image acquired by the imaging arrangement. Using a mobile C-arm X-ray system as an example, the parameters and settings of the imaging arrangement may include peak kilovoltage (kVp) and exposure parameter (mAs). An X-ray image may be acquired based on these parameters and settings.

The control signal indicative of a mode change may be obtained in different manners.

In an example, the control signal may be obtained from the user interface 70. For example, the user interface may comprise a button, a navigation menu, and the like to allow an operator for switching the user interface between the control mode and the moving assistance mode. For example, during the surgery, the C-arm 16 may move around a patient lying on the operation table. There is a risk that the moving C-arm may collide against OT equipment. In this case, the operator may switch the user interface from the control mode for controlling the setting and operation of the imaging arrangement to the moving assistance mode with a camera image showing a clear view of e.g. an area below the operating table for easing the placement of the C-arm 16. After the placement, the operator may switch the user interface from the moving assistance mode to the control mode to continue controlling the setting and operation of the imaging arrangement.

In another example, the control signal may be configured to be generated in response to a change of power state of the imaging arrangement 10. For example, after an imaging procedure, the operator may switch off the imaging arrangement and want to move the mobile imaging system out of the OT. As the imaging arrangement is powered off, i.e. a change of power state of the imaging arrangement from an on-state to an off-state, the user interface may automatically be switched, by the controller 60, from the control mode to the moving assistance mode. In other words, the user interface 70 may automatically display a camera image detected by the camera arrangement 50. This may allow an operator to have a better view of e.g. the blind spots in front of the mobile stand, when moving the mobile imaging system out of the OT. Accordingly, collision may be avoided. On the other hand, during the placement of the imaging arrangement, the user interface may be in the moving assistance mode with a camera image that covers an area below the operating table for easing the placement of the imaging arrangement. After the placement, the operator may switch on the imaging arrangement, i.e. a change of power state of the imaging arrangement from an off-state to an on-state, the user interface may automatically be switched, by the controller 60, from the moving assistance mode to the control mode allowing the operator to control the setting and operation of the imaging arrangement.

Optionally, the mobile C-arm X-ray system 100 may further comprise an electromagnetic brake (not shown) to bring the moving stand 40 and/or the C-arm 16 to a halt. The electromagnetic brake may be designed in such a manner that the brake is released, when at least one camera in the camera arrangement is on and/or the user interface is in the moving assistance mode. In other words, the mobile stand 40 may be allowed to travel, if the camera image is displayed showing the region in front of the mobile stand for assisting in moving the mobile stand 40 and/or the C-arm 16. The electromagnetic brake may be activated, if an alert signal representative of a presence of an obstacle within a predefined distance range is received. For example, the alert signal may be generated, if an obstacle is detected within a high-risk space, e.g. indicated by a red color 32 shown in Fig. 2. Optionally, the electromagnetic brake may be activated, if the camera arrangement is off. In this case, as no camera image is displayed for assisting in moving the mobile stand 40, the electromagnetic brake may bring the movement of the mobile stand 40 and/or the C-arm 16 to a halt for avoiding a collision. Additionally, due to manual movement required in power fail condition, the minimum force requirement as per IEC medical standards ,which makes break mechanism more complex for all the axis. This can be avoided using battery power to power the electromagnetic brake.

Fig. 3 schematically illustrates a block diagram of a further exemplary mobile imaging system 100. The exemplary mobile imaging system 100 in Fig. 3 is a mobile X-ray system. One skilled in the art would appreciate that the mobile imaging system may be any other mobile systems, such as mobile ultrasound system and mobile patient monitoring system.

When the imaging arrangement 10 is on, the user interface 70 is used to view X-ray images and to control X-ray parameters and settings. In this example, the imaging arrangement 10 comprises an X-ray tube 12, an X-ray generator 18, and an X-ray control unit 19. To achieve a higher dose, the mobile X-ray system may use a battery pack 20 of a higher capacity and has a cut-off voltage to avoid dead drain. A battery charger unit 22 may be provided to put the energy from an external power source 21 to charge the battery pack 20 until it reaches a specific voltage and then it trickle charges the battery pack 20 until it is disconnected. When the imaging arrangement 10 is on, a relay logic R1 will be open and camera monitoring logic circuits, such as a DC-DC converter 82 and a control logic 86, will be disabled. The relay logic R2 will provide a normal power to a stand UI programmable computer (PC) 84. There is a communication between the X-ray control unit 19 and the stand UI PC 84, the user can select a control mode or a moving assistance mode via the user interface 70. This will be also described hereafter in conjunction with Fig. 5.

The same battery pack 20 is also used for the X-ray generation system to power up a camera monitoring system 80. When the imaging arrangement, such as the X-ray generation system in Fig. 3 is switched off, the battery pack 20 holds a good amount of power. The relay logic R1 is closed and the DC-DC converter 82 is connected to the battery pack 20. The same low voltage is also used by the control logic 86 to switch the relay logic R2 for a pre-defined time. Thus, the stand UI PC 84 will be supplied with power and the user interface 70 will become live for a pre-defined time, such as 10 minutes. As there is no communication with the X-ray control logic 19, a control of parameters and settings of the imaging arrangement is disabled. Therefore, the user only has the option to view the camera image. The operator can thus view a live camera image sent by the camera arrangement 50 and move the mobile X-ray system 100 or place the C-arm 16. In other words, the user interface 70 is switched from the control mode to the moving assistance mode. After the pre-defined time, the relay logic R2 will cut off power of the stand UI PC 84. Therefore, the user interface 70 does not further consume the power from the battery pack 20. If the operator wants to view the camera image, the operator may use e.g. an ON/OFF key toggle 88 to switch on the user interface for a pre-defined time. Although the camera arrangement 30 illustrated in Fig. 3 comprises a wide-angle night version camera, there is possibility to connect multiple cameras to the system for better viability and the user may select one of more cameras for viewing. This will be also described hereafter in conjunction with Fig. 6.

According to a second aspect of the present disclosure, there is provided a computer-implemented method 200 for controlling mobile imaging system that comprises an imaging arrangement for acquiring image data, an internal power source for supplying power to the mobile imaging system, a display arrangement with at least one display device; a mobile stand bearing the imaging arrangement, the internal power source, and the display arrangement, and a camera arrangement with at least one camera arranged on a frond end of the mobile stand to have a field of view that covers a region in front of the mobile stand. The method comprises:
- receiving 210, by a controller, a control signal indicative of a mode change; and
- controlling 220, by the controller, a user interface to switch between a moving assistance mode and a control mode according to the control signal;
wherein, in the moving assistance mode, the user interface receives a signal from the camera arrangement and displays a camera image showing the region in front of the mobile stand; and
wherein, in the control mode, the user interface enables a control of parameters and settings of the imaging arrangement for acquiring image data.

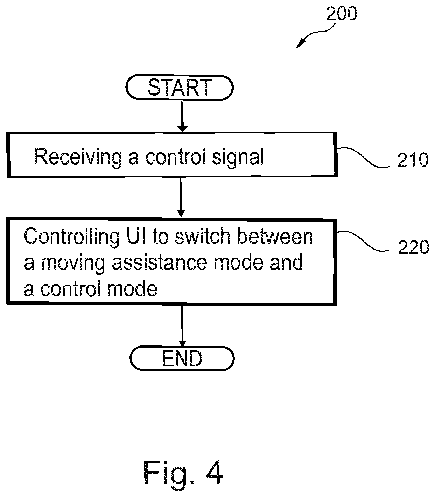

Fig. 4 illustrates an example of the computer-implemented method 200 according to the second aspect of the present disclosure.

The computer-implemented method 200 may be implemented as a device, module or related component in a set of logic instructions stored in a non-transitory machine- or computer-readable storage medium such as random access memory (RAM), read only memory (ROM), programmable ROM (PROM), firmware, flash memory, etc., in configurable logic such as, for example, programmable logic arrays (PLAs), field programmable gate arrays (FPGAs), complex programmable logic devices (CPLDs), in fixed-functionality hardware logic using circuit technology such as, for example, application specific integrated circuit (ASIC), complementary metal oxide semiconductor (CMOS) or transistor-transistor logic (TTL) technology, or any combination thereof. For example, computer program code to carry out operations shown in the method 200 may be written in any combination of one or more programming languages, including an object oriented programming language such as JAVA, SMALLTALK, C++, Python, or the like and conventional procedural programming languages, such as the "C" programming language or similar programming languages.

In an example, the control signal indicative of a mode change may be received from the user interface. This example will be described hereafter in conjunction with Fig. 5.

Fig. 5 is a flowchart illustrating a situation, where the mobile X-ray system of Fig. 3 is switched on. In the following, the battery is provided as an example of an internal power source. However, one skilled in the art would appreciate that other internal power sources, such as fuel cells, can also be used.
In step a1), the mobile X-ray system is switched on.
In step b1), the battery charger unit 20 charges the battery pack 20 until it reaches a specific voltage.
In step c1), the X-ray control logic 19 is on.
In step d1), the relay logic R2 provides a normal power to the stand UI PC 84. In step e1), there is a communication between the X-ray control unit 19 and the stand UI PC 84. A control of X-ray parameters and settings is available on the user interface 70.
In step f1), the user can select, via the user interface, a camera for system placement, which then generates a control signal indicative of a mode change.
In step g1), the user interface 70 now switches from the control mode to a moving assistance mode. In the moving assistance mode, the user interface 70 shows a camera image of an area in front of the mobile stand for easing the placement of the C-arm 16.
In step h1), after the placement, the user may switch the user interface from the moving assistance mode back to the control mode. In the control mode, the user may control the settings and operation of the X-ray generation system for acquiring image data.
In step i1), after inputting the parameters and settings of the X-ray generation system, the system is ready for exposure.

In an example, the control signal indicative of a mode change may be received in response to change of power state of the imaging arrangement. This example will be described hereafter in conjunction with Fig. 6.

Fig. 6 is a flowchart illustrating a situation, where the mobile X-ray system of Fig. 3 is switched off. In the following, the battery is provided as an example of an internal power source. However, one skilled in the art would appreciate that other internal power sources, such as fuel cells, can also be used.
In step a2), the mobile X-ray system is switched off.
In step b2), when the X-ray generation system of Fig. 3 is switched off, the relay logic R1 is closed.
In step c2), the DC-DC converter 82 is connected to the battery pack 20.
In step d2), the same low voltage is used by the control logic 86 to switch the relay logic R2 for a pre-defined time. Thus, the stand UI PC 84 will be supplied with power and the user interface 70 will become live for a pre-defined time, such as 10 minutes.
In step e2), as there is no communication with the X-ray control logic 19, a control of parameters and settings of the imaging arrangement is disabled. Therefore, the user only has the option to view the camera image.
In step f2), if the user interface 70 is live, the user can place the system or transport the system with the aid of the camera image displayed on the screen, i.e. step g2). After the pre-defined time, the user interface 70 is automatically switched off, i.e. step i2).

On the other hand, if the user interface 70 is off, and the operator wants to view the camera image, the operator may use e.g. an ON/OFF key toggle 88 to switch on the user interface for a pre-defined time, i.e. step h2).

It should also be understood that, unless clearly indicated to the contrary, in any methods claimed herein that include more than one step or act, the order of the steps or acts of the method is not necessarily limited to the order in which the steps or acts of the method are recited.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A mobile imaging system (100), comprising:
- an imaging arrangement (10) for acquiring image data;
- an internal power source (20) for supplying power to the mobile imaging system;
- a display arrangement (30) with at least one display device;
- a mobile stand (40) bearing the imaging arrangement, the internal power source, and the display arrangement;
- a camera arrangement (50) with at least one camera arranged on a frond end of the mobile stand to have a field of view that covers a region in front of the mobile stand; and
- a controller (60) configured to receive a control signal indicative of a mode change and to control a user interface (70) displayed on the at least one display device to switch between a moving assistance mode and a control mode according to the control signal;
wherein, in the moving assistance mode, the user interface is configured to receive a signal from the camera arrangement and to display a camera image showing the region in front of the mobile stand for assisting in moving the mobile stand; and
wherein, in the control mode, the user interface is configured to enable a control of parameters and settings of the imaging arrangement for acquiring image data or a display of an image acquired by the imaging arrangement.

2. Mobile imaging system according to claim 1,
wherein the control signal is obtainable from the user interface.

3. Mobile imaging system according to claim 1 or 2,
wherein the control signal is configured to be generated in response to a change of power state of the imaging arrangement.

4. Mobile imaging system according to claim 3,
wherein, the control signal is configured to be generated to switch the user interface to the moving assistance mode, when a power state of the imaging arrangement is changed from an on-state to an off-state.

5. Mobile imaging system according to any one of the preceding claims,
wherein, in the moving assistance mode, the controller is configured to receive a signal indicative of distance information in front of the mobile stand and/or height information in front of the mobile stand; and
wherein, the user interface is configured to display the camera image together with a distance marking indicative of the distance information projected onto a ground and/or a height marking indicative of the height information.

6. Mobile imaging system according to any of the preceding claims,
wherein, the display arrangement is configured to be powered by the internal power source for a predefined time, when a power state of the imaging arrangement is an off-state.

7. Mobile imaging system according to any one of the preceding claims,
wherein, the display arrangement comprises:
(i) a single display device with an image screen for displaying the camera image, and the parameters and settings of the imaging arrangement; or
(ii) at least two display devices with at least two image screens for displaying the camera image, and the parameters and settings of the imaging arrangement.

8. Mobile imaging system according to any one of the preceding claims,
wherein the display arrangement is arranged on a back end of the mobile stand.

9. Mobile imaging system according to any one of the preceding claims,
wherein, the camera arrangement is configured to be supplied with power from the internal power source .

10. Mobile imaging system according to any of the preceding claims,
wherein, the camera arrangement comprises a wide-angle night version camera.

11. Mobile imaging system according to any one of the preceding claims, further comprising:
- an electromagnetic brake configured to be released when at least one camera in the camera arrangement is on and/or the user interface is in the moving assistance mode, as long as any alert signal representative of a presence of an obstacle within a predefined distance range is not received.

12. Mobile imaging system according to any one of the preceding claims,
wherein, the imaging arrangement comprises at least one of X-ray, ultrasound, or patient monitoring device.

13. A computer-implemented method (200) for controlling a mobile imaging system that comprises an imaging arrangement for acquiring image data, an internal power source for supplying power to the mobile imaging system, a display arrangement with at least one display device; a mobile stand bearing the imaging arrangement, the internal power source, and the display arrangement, and a camera arrangement with at least one camera arranged on a frond end of the mobile stand to have a field of view that covers a region in front of the mobile stand, the method comprising:
- receiving (210), by a controller, a control signal indicative of a mode change; and
- controlling (220), by the controller, a user interface to switch between a moving assistance mode and a control mode according to the control signal;
wherein, in the moving assistance mode, the user interface receives a signal from the camera arrangement and displays a camera image showing the region in front of the mobile stand; and
wherein, in the control mode, the user interface enables a control of parameters and settings of the imaging arrangement for acquiring image data.

14. A computer program element for controlling a mobile imaging system according to any one of claims 1 to 12, which when being executed by a processor is configured to carry out the method according to claim 13.

15. A computer readable medium having stored thereon the program element of claim 14.
